(19) Europäisches Patentamt
European Patent Office
Office européen des brevets

(11) Veröffentlichungsnummer: 0 372 581
A2

# (12) EUROPÄISCHE PATENTANMELDUNG

(21) Anmeldenummer: 89122710.0

(22) Anmeldetag: 08.12.89

(51) Int. Cl.5: G01N 33/543, G01N 33/548, G01N 33/74, G01N 33/82

(30) Priorität: 09.12.88 DE 3841566

(43) Veröffentlichungstag der Anmeldung: 13.06.90 Patentblatt 90/24

(84) Benannte Vertragsstaaten: AT BE CH DE ES FR GB GR IT LI LU NL SE

(71) Anmelder: BOEHRINGER MANNHEIM GMBH
Sandhofer Strasse 112-132
D-6800 Mannheim-Waldhof(DE)

(72) Erfinder: Mang, Thomas, Prof. Dr. rer. nat.
Saalangerstrasse 40
D-8122 Penzberg(DE)
Erfinder: Maier, Josef
Schmädlstrasse 15
D-8120 Weilheim(DE)

(74) Vertreter: Huber, Bernhard, Dipl.-Chem. et al
Möhlstrasse 22 Postfach 860 820
D-8000 München 86(DE)

## (54) Verfahren zur Herstellung einer Festphasenmatrix, an die Haptene kovalent gebunden sind.

(57) Zur Herstellung einer Festphasenmatrix, an die Haptene kovalent gebunden sind, bindet man das Hapten an dem als Festphase dienenden Material direkt ohne Bindung eines Spacers kovalent durch Umsetzen des mindestens eine funktionelle Gruppe enthaltenden Haptens an ein Festphasenmaterial, wobei eine der beiden Komponenten vorher aktiviert wurde.

EP 0 372 581 A2

## Verfahren zur Herstellung einer Festphasenmatrix, an die Haptene kovalent gebunden sind

Trägermaterialien, an die Haptene adsorptiv oder kovalent gebunden sind, werden sowohl bei der Durchführung immunologischer Nachweisverfahren nach dem Prinzip des Immunoassays als auch für die Affinitäts-Chromatographie verwendet. Dazu ist es erforderlich, daß die Bindung der Haptene an die Matrix so stark ist, daß sie unter den in der Affinitäts-Chromatographie bzw. bei der Durchführung des Immunoassays angewandten Bedingungen nicht abgelöst werden.

Bei der Affinitätschromatographie werden Haptene an einen Träger gebunden, um mit diesem Hapten spezifisch bindefähige Substanzen, wie z. B. gegen das Hapten gerichtete Antikörper, aus einem Gemisch abzutrennen. Nach der Auftrennung des Gemisches z.B. in einer Chromatographiesäule muß die gebundene spezifisch bindefähige Substanz wieder vom Hapten eluiert werden. Dabei darf aber das Hapten nicht abgelöst werden.

Bei Immunoassays dient das an eine feste Phase gebundene Hapten dazu, einen zu bestimmenden Komplex aus einer Reaktionslösung abzutrennen durch Bindung mit einer mit dem Hapten spezifisch bindefähigen Substanz, wie z.B. einem gegen das Hapten gerichteten Antikörper. Auch hier ist es wesentlich, daß das Hapten an dem Träger gebunden bleibt, da ansonsten die Ergebnisse verfälscht werden.

Die bisher bekannten Verfahren zur Bindung von Haptenen an eine Matrix erwiesen sich alle als nicht optimal. Die Bindung der Haptene an die Matrix war nicht ausreichend stark, so daß der Träger bei Anwendung der für die einzelnen Verfahren notwendigen Bedingungen ausblutete, d. h. das Hapten abgab.

Ein anderes Problem bei der Bindung des Haptens an einen Träger besteht darin, daß das Hapten so gebunden werden muß, daß seine aktive Stelle nicht blockiert wird, so daß seine Aktivität auch nicht teilweise verlorengeht. Bisher wurde es daher für erforderlich gehalten, Haptene über einen Spacer, der mindestens 4, besser 6 oder mehr C-Atome aufwies, zu binden. So ist beispielsweise in einer Firmenbroschüre der Firma Pharmacia, "Affinity Chromatography principles & methods", auf Seite 19 unter Punkt 2 angegeben, daß für Liganden mit niedrigem Molekulargewicht die Verwendung eines Spacers notwendig ist, um seine Zugänglichkeit gegenüber den mit ihm reagierenden Molekülen zu gewährleisten. Dasselbe ist nochmals auf Seite 20 unter 2.2 angegeben. Unter 2.3 wird als optimale Spacer-Armlänge ein Spacer mit 6 Kohlenstoffatomen angegeben. Trotzdem konnten mit diesen über Spacer festphasengebundenen Haptene keine optimalen Ergebnisse erzielt werden. Insbesondere läßt die Stabilität noch zu wünschen übrig, so daß eine längere Lagerung derartiger Festphasenmatrizes nicht möglich ist.

Es war daher Aufgabe der vorliegenden Erfindung, ein Verfahren zur Verfügung zu stellen, mit dem Haptene so an einen Träger fixiert werden können, daß sie ihre Aktivität nicht verlieren, daß sie bei Anwendung der bei Immunoassays oder Affinitätschromatographie erforderlichen Bedingungen nicht von dem Träger abgelöst werden und daß sie längere Zeit ohne Qualitätsverlust gelagert werden können.

Diese Aufgabe wird gelöst durch ein Verfahren zur Herstellung einer Festphasenmatrix, an die Haptene kovalent gebunden sind, welches dadurch gekennzeichnet ist, daß man durch Umsetzen eines mindestens eine funktionelle Gruppe enthaltenden Haptens und eines Festphasenmaterials, wobei eine der beiden Komponenten vorher aktiviert wurde, das Hapten an dem als Festphase dienenden Material direkt ohne Bindung eines Spacers kovalent bindet.

Überraschenderweise wurde festgestellt, daß bei Anwendung des erfindungsgemäßen Verfahrens das Hapten einerseits so stark an die Matrix gebunden wird, daß es auch bei Anwendung drastischer Bedingungen nicht abgelöst wird und andererseits auch bei längerer Lagerung gebunden bleibt, ohne daß es seine Aktivität im wesentlichen verliert.

Das erfindungsgemäße Verfahren dient zur Herstellung einer haptenisierten Festphasenmatrix. Als Festphasenmatrix kann jedes Material verwendet werden, das für die Bindung mit dem Hapten geeignete funktionelle Gruppen wie z.B. Hydroxy-, Carboxyl-, Aldehyd- oder Aminogruppen aufweist. Geeignet sind hierzu Polymere und polysaccharidhaltige Materialien. Bevorzugt wird als Festphasenmaterial wasserunlösliches polysaccharidhaltiges Material verwendet, das gegebenenfalls mit inerten Bestandteilen vermischt sein kann. Besonders bevorzugt werden cellulosehaltige Materialien eingesetzt, insbesondere reine Cellulose, Sulfitzellstoff, Zellwolle und/oder Celluloseacetat sowie Mischungen dieser Komponenten. Das cellulosehaltige Material kann auch mit anderen Fasern, die in solchen Materialien üblicherweise verwendet werden, vermischt werden. Geeignet sind beispielsweise Polyester, Nylon oder Polyacrylnitril.

An die Festphasenmatrix wird ein Hapten gebunden. Haptene sind definitionsgemäß Moleküle, die ein Molekulargewicht von weniger als ca. 1.500 Dalton aufweisen, allein nicht in der Lage sind, eine Immunantwort auszulösen, jedoch mit einem Antikörper reagieren können. Erfindungsgemäß können solche Haptene verwendet werden, die mindestens eine funktionelle Gruppen aufweisen. Diese funktionelle Gruppe ist

bevorzugt eine Amino-, Hydroxy-, Aldehyd- oder Carboxylgruppe. Besonders geeignet ist das erfindungsgemäße Verfahren zur Bindung von Hormonen wie Cortison, Oestrol, Oestriol, Oestradiol, Testosteron, Progesteron, Aldosteron, $T_3$ und $T_4$; Vitaminen wie dem Vitamin B12 oder Folat sowie Arzneimitteln wie Digoxin, Digitoxin, Digoxigenin, Phenobarbital oder Theophyllin und zur Bindung von Biotin.

Zur Kupplung des Haptens an die Festphase wird entweder das Hapten oder das Festphasenmaterial aktiviert. Verfahren hierzu sind dem Fachmann bekannt. Beispielsweise geeignet ist die Umwandlung in einen aktivierten Ester oder ein Imidazolid. Möglichkeiten zur Aktivierung sind beispielsweise beschrieben in K. Lübke, E. Schröder, G. Kloss, Chemie und Biochemie der Aminosäuren, Peptide und Proteine I, G. Thieme Verlag, Stuttgart, 1975, S. 136 bis 137.

Das Festphasenmaterial und das Hapten werden bevorzugt in wasserfreiem Medium umgesetzt. Die Umsetzung erfolgt dabei vorzugsweise in wasserfreiem organischem Lösungsmittel wie Aceton, DMF oder Dioxan.

Die erfindungsgemäß hergestellte Festphasenmatrix, an die die Haptene kovalent gebunden sind, wird in an sich bekannter Weise je nach Verwendungszweck verarbeitet. Für den Einsatz in der Affinitätschromatographie liegt das Material bevorzugt in Form von Kugeln oder Pulver vor. Für den Einsatz in Immunoassays wird das Material bevorzugt in Form von Filmen, Vliesen oder Papier verwendet. Dabei ist es einerseits möglich, das Festphasenmaterial zuerst mit dem Hapten umzusetzen und anschließend das haptenisierte Material in die gewünschte Form zu bringen. Ebenso möglich ist es, zuerst das Festphasenmaterial in die gewünschte Form zu bringen und anschließend mit dem Hapten umzusetzen. So können beispielsweise zuerst Cellulosefasern haptenisiert werden und anschließend dann die Fasern zu einem Vlies verarbeitet werden. Andererseits können zuerst aus dem Festphasenmaterial Kugeln geformt werden, die dann anschließend haptenisiert werden.

Die Umsetzung des Festphasenmaterials mit dem Hapten erfolgt bevorzugt in einer Vorrichtung, die gekennzeichnet ist durch einen Wickelkern in einem Behälter für Reaktionslösung, welcher ein in seiner Wand mit einer Vielzahl von Öffnungen versehenes, an einem Ende verschlossenes Rohr für die Aufnahme eines Wickels des zu behandelnden Materials und zwei axiale Wangenscheiben zur Abdichtung der axialen Stirnseiten des Wickels aufweist und durch einen Umwälzpumpenkreis, der die Reaktionslösung aus dem Behälter dem anderen Ende des Rohrs zuführt.

Bevorzugt sind dabei die Wangenscheiben gegen die axialen Stirnseiten des Wickels spannbar.

Bevorzugt wird nun in den Behälter die das aktivierte Hapten enthaltende Reaktionslösung eingefüllt. Das Festphasenmaterial, vorzugsweise Papier, das bahnenförmig vorliegt, wird auf den Wickelkern aufgewickelt und mit einer Gaze fixiert. Der Papierrand wird durch die Wangenscheiben abgedichtet. Die Reaktionslösung wird dann über den Umwälzpumpenkreis von dem Behälter dem anderen Ende des Rohrs zugeführt. Dabei durchströmt die Reaktionslösung das aufgewickelte Material nach allen Richtungen radial von innen nach außen. Vorteil dieser Vorrichtung ist es, daß nur wenig organisches Lösungsmittel verwendet werden muß. Weiterhin ist die Apparatur sehr leicht zu handhaben und zu reinigen.

Mit dem erfindungsgemäßen Verfahren kann unter Verwendung leicht verfügbarer Einsatzstoffe eine vielseitig verwendbare Festphasenmatrix im Produktionsmaßstab homogen und reproduzierbar hergestellt werden, die sowohl für die Chromatographie als auch z.B. als spezifische Matrix oder als Universalmatrix in Immunoassays eingesetzt werden kann. Die erfindungsgemäß erhaltene Festphasenmatrix weist eine sehr gute Stabilität auf.

Die Erfindung wird durch die Figuren und durch die Beispiele erläutert.

Fig. 1 zeigt eine Vorrichtung zur Durchführung des erfindungsgemäßen Verfahrens.

Fig. 2 zeigt ein Einsatzelement für einen Zentrifugalanalysator.

Fig. 1 zeigt einen Behälter für Reaktionslösung 1, in dem sich ein Wickelkern 3 befindet. Der Wickelkern weist ein an einem Ende 9 verschlossenes Rohr 11 für die Aufnahme eines Wickels des zu behandelnden Materials auf, wobei das Rohr 11 eine Vielzahl von Öffnungen 7 aufweist. An den axialen Stirnseiten des Wickels sind zwei axiale Wangenscheiben 15, 17 zur Abdichtung angeordnet, die gegen die axialen Stirnseiten des Wickels spannbar sind. Die Vorrichtung weist weiterhin einen Umwälzpumpenkreis 19 auf, der die Reaktionslösung aus dem Behälter 1 dem anderen Ende (21) des Rohrs 11 zuführt.

**Beispiel 1**

Kupplung von BOC-$T_3$-Imidazolid an Cellulose

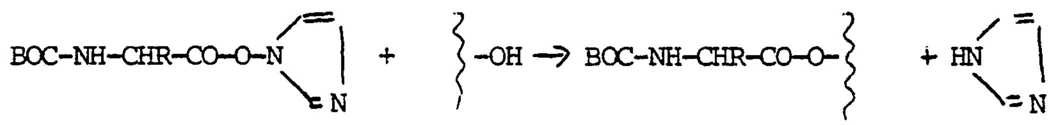

Zu 2 g Papier (Cellulose-Linters) wurden 5 mg BOC-$T_3$-Imidazolid in 125 ml trockenem Aceton gegeben und über Nacht bei Raumtemperatur geschüttelt. Anschließend wurde viermal mit Aceton und zweimal mit Wasser gewaschen. Das so behandelte Papier wurde über 12 Stunden mit 2,5 1 20 mmol/l Phosphatpuffer mit pH 8 in einer Säule unter langsamem Durchspülen behandelt, anschließend mit je 50 ml $H_2O$ und Aceton behandelt und im Vakuum getrocknet.

**Beispiel 2**

Aktivierung der Cellulose mit Tosylchlorid und Kopplung an $T_3$.

Zu 2 g Papier wurden 200 mg Tosylchlorid in 50 ml trockenem Dioxan gegeben. Nach Zugabe von 20 µl Pyridin wurde eine Stunde geschüttelt und das Papier mit 2x50 ml Dioxan und 2x50 ml $H_2O$ gewaschen.

Zu 2 g Papier wurde eine Lösung aus 10 mg $T_3$ in 0,1 mol/l $NaHCO_3$ gegeben und unter Schütteln 24 Stunden inkubiert. Anschließend wurde mit je 50 ml 0,1 mol/l NaCl, Wasser und mit 0,1 mol/l Phosphat-Puffer, pH 7,5, und Wasser gewaschen und im Vakuum getrocknet.

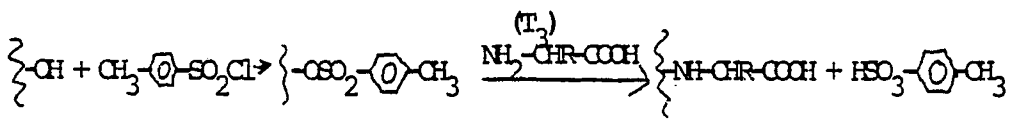


**Beispiel 3** (Vergleich)

Zum Vergleich wurde an Papier synthetisches Polyhapten über einen hochmolekularen Spacer gebunden. Davon wurde zuerst Allylamin mit Acrylamid copolymerisiert. Eine Lösung, bestehend aus 200 ml $H_2O$ bidestilliert, 15 g Acrylamid (Serva, viermal kristallisiert) und 0,45 ml Allylamin, wurde mit 2 ml einer 10%igen Starterlösung versetzt, leicht gerührt und bis zum Einsetzen der Polymerisation mäßig mit Stickstoff durchlüftet. Nach 12 Stunden Nachreaktionszeit wurde 24 Stunden gegen $H_2O$ dialysiert. Das Copolymerisat wurde anschließend lyophilisiert und stand für die weitere Aufarbeitung zur Verfügung.

An das Copolymerisat wurden $T_3$ und Digoxigenin als Haptene gekuppelt.

I. $T_3$

1,6 g des erhaltenen Lyophilisats wurden in 160 ml $KPO_4$, 10 mmol/l, pH 8,2, gelöst und langsam mit 80 ml Dioxan versetzt. In die klare Lösung wurden 100 mg BOC-$T_3$-N-Hydroxysuccinimid/20 ml Dioxan zugetropft. Die Reaktionslösung wurde 12 Stunden bei 20° C unter Lichtschutz stehen gelassen. Zur Aufreinigung wurde das Reaktionsprodukt in 1000 ml Aceton gefällt und abzentrifugiert (Stock-Zentrifuge, eine Stunde, 3000 UPM). Der Niederschlag wurde in 160 ml bidestilliertem $H_2O$ aufgenommen und erneut mit 500 ml Aceton gefällt. Nach Zentrifugation wurde das so aufgereinigte Polyhapten in 80 ml $H_2O$ gelöst und bei -20° C gelagert.

II. Digoxigenin

Das Verfahren wurde analog zu I) mit veränderten Konzentrationen der Einsatzstoffe durchgeführt.

1,6 g Co-Polymerisat, 50 ml 10 mmol/l $KPO_4$, pH 8,2, 15 ml Dioxan, 32 mg Digoxigenin-succinyl-N-Hydroxysuccinimid/5 ml Dioxan.

Die Haptene werden an Papier als Trägermaterial gebunden. Das Papier wurde vor der Kupplung aktiviert.

Methode a), alkalisierte Cellulose, aktiviert mit Trichlortriazin (TCT).

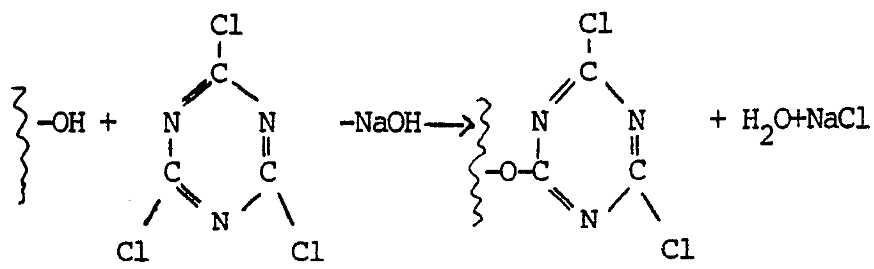


Geräte: Reaktor nach Fig. 1, 5 x 10 cm
Kern: 1,5 x 10 cm
Pumpe: Verder Zahnradpumpe, Vor- und Rücklauf.

1,6 m (Schleicher und Schull S 3512) wurden mit Polyethylen-Stützgaze um den Kern gewickelt und in den Reaktor gegeben. Danach wurde eine Stunde mit 500 ml 50 mmol/l methanolischer NaOH umgepumpt (500 ml/min). Nach Beendigung der Alkalisierung wurde viermal mit 400 ml Aceton p. a. gespült und eine Stunde mit 500 ml einer 5%igen TCT/Aceton-Lösung umgepumpt. Anschließend wurde sechsmal mit 400 ml Aceton gespült. Die Waschlösung wurde mit 4-(4-Nitrobenzyl)-pyridin auf freies TCT getestet. Das aktivierte Papier wurde aus dem Reaktor genommen, getrocknet und stand für den weiteren Verarbeitungsprozeß zur Verfügung.
Lagerbedingungen: 4° C, trocken, maximal 2 Wochen.

Methode b)

Aktivierung unter Zugabe von Diisopropylethylamin mit TCT

Das Verfahren wurde analog Methode a) durchgeführt, jedoch ohne vorherige Alkalisierung. Es wurden 500 ml 1%ige TCT/Aceton-Lösung und 5 ml Diisopropylethylamin verwendet. Die Reaktionszeit betrug 6 Stunden. Die Waschprozedur erfolgte, wie in Methode a) beschrieben.

Methode c)

Perjodat-Aktivierung des Papierträgers

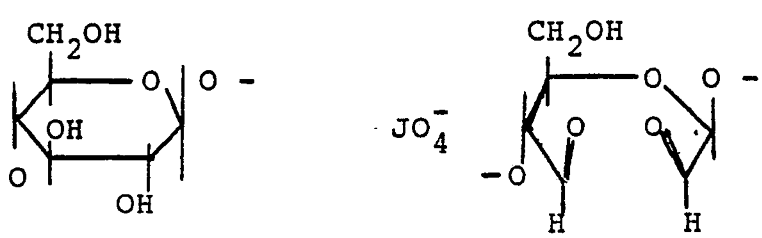


Zu 10 g Papier wurde 1 ℓ einer 0,1 mol/l $NaJO_4^-$ -Lösung gegeben und bei Raumtemperatur über Nacht geschüttelt. Anschließend wurde mit $H_2O$ so lange nachgewaschen, bis mit KJ-Stärkepapier keine Verfärbung desselben mehr sichtbar war. Dann Trocknung im Vakuum bei Raumtemperatur.

An die gemäß Methoden a) bis c) erhaltenen Papierträger wurden die Polyhaptene I und II gekuppelt.

α) Bindung an den TCT-aktivierten Papierträger

1,6 m TCT-Papier (Beispiel 1b) wurden 12 Stunden bei Raumtemperatur mit einer Lösung aus 320 mg $T_3$ (Beispiel 3l) in 300 ml 0,2 mol/1 $KPO_4$, pH 8,2, beladen. Anschließend wurde 5 Stunden mit 400 ml 0,5 mol/l Ethanolamin-Lösung, pH 8,2, mit einer Flußrate von 300 ml/min umgepumpt.

Dann wurde wie folgt gewaschen: 20 1 50 mmol/l Acetat, pH 6,0, 5 o/oo Tween, 20 l.
Pumpzeit: 12 Stunden.
20 l 0,1 mol/l $KPO_4$/0,5 mol/l NaCl, 5 o/oo Tween 20, pH 8,2
Pumpzeit: 12 Stunden.
10 l 0,5 mol/l $KPO_4$, pH 6,0, 5 o/oo Tween 20 Pumpzeit: 8 Stunden.
5 l 1 mmol/l $KPO_4$, pH 6,0 Pumpzeit: 2 Stunden.
6x500 ml Aceton p.a.
Pumpgeschwindigkeit: 300 ml/min.

Nach dem Waschprozeß wurden die Papiere dem Reaktor entnommen und 8 Stunden im Vakuum bei $10^{-2}$ Torr getrocknet.

ß) Bindung des $T_3$-Polymerisats an Perjodat-aktivierten Papierträger

5 g perjodatoxidiertes Cellulosepapier wurden 12 Stunden bei Raumtemperatur mit einer Lösung aus 18,5 mg T3-Polymerisat (siehe Beispiel 5) und 0,94 g $NaBH_3CN$ in 250 ml 0,1 mol/l $KPO_4$, pH 7,2 beladen. Flußrate: 300 ml/min.

Waschung und Trocknung erfolgten wie bei α).

**Beispiel** 4 (Vergleich)

Weiterhin wurde zum Vergleich an Trichlortriazin (TCT)-aktivierten Papierträger, der gemäß Beispiel 3 erhalten wurde, Hapten über einen niedermolekularen Spacer gekuppelt.

1 g TCT-aktiviertes Papier wurde mit einer Lösung von 5 bis 10 µl 1,8-Diamino-3,5-dioxaoctan (DAD00) in 125 ml Dioxan versetzt und eine Stunde geschüttelt. Anschließend wurde viermal mit Dioxan gewaschen.

Zu dem so behandelten Papier wurde eine Lösung von 10 mg BOC[1)]-$T_3$-OSu[2)] in 125 ml Dioxan gegeben und 2 Stunden geschüttelt.

Weiterhin wurde 1 g TCT-aktiviertes Papier mit 1 bis 5 mg DADOO-BOC-$T_3$ in 125 ml Dioxan 2 Stunden geschüttelt und dann viermal mit Dioxan behandelt.

Danach wurde jeweils dreimal mit Aceton und $H_2O$ gewaschen, anschließend mit 20 mmol/l Acetat, pH 6 und 0,1 % Tween 20 12 Stunden in einer Säule unter langsamem Durchspülen behandelt. Nach Waschen mit $H_2O$ wurde das Papier zweimal mit 50 ml Aceton behandelt und im Vakuum getrocknet.

Waschprozedur erfolgte wie oben. [1)] tert.-Butyloxycarbonyl
[2)] N-Hydroxysuccinimid

**Beispiel 5**

Es wurde die Stabilität der gemäß den Beispielen 1 bis 4 erhaltenen Festphasenmatrizes verglichen. Dazu wurden die Matrizes jeweils drei Wochen bei 37°C im Trockenschrank gelagert. Die Bestimmung des Bindekapazitätsabfalls nach Belastung erfolgte unter Verwendung eines Einsatzelements gemäß Fig. 2 in einem Gerät gem. EP-A 0167171 und 0167175.

Bestimmung des Einsatzelements:

a: Vlies aus 40% Linters 941, 50% Polyamid, 10% Kuralon (Polyvinylalkoholfaser, Fa. Kuraray, Japan)
30 mmol/l $Na_3PO_4$ x $12H_2O$
0,5 o/oo Genapol PF20 (Hoechst AG, Alkylpolyglycolether mit Ethylenoxid-Propylenoxid-Blockpolymeren)

b: Leerkammer (kein Vlies)

c: Vlies aus 20% Linters 941, 30% Zellwolle,
40% Polyamid 8,2/4, 10% Kuralon,

getränkt mit einer Lösung von:
38 mmol/l $Na_2H\ PO_4 \times 2\ H_2O$
36 mmol/l $Na\ H_2\ PO_4 \times H_2O$
3,5 mmol/l $MgK_2$ EDTA x 2 $H_2O$
0,7% Rinderserumalbumin
0,7% Crotein C
0,035% Anilinonaphtylsulfonsäure (ANS)
0,01% Genapol PF 20

d: Vlies aus 80% Polyester, 20% Zellwolle getränkt mit einer Lösung von:
280 U/l polyklonale Antikörper gegen $T_3$, gekoppelt an ß-Galactosidase. 100 mmol/l 4-(2-Hydroxyethyl)-1-piperazinethanolsulfonsäure (HEPES) pH 7,25
5 mmol/l Mg-L-Aspartat
1% Crotein C
zur Lagerung wird das getränkte Vlies lyophilisiert.

e: Vlies analog Beispiel 1 (Matrix) f: Vlies aus 20% Linters, 30 % Zellwolle,
40 % Polyamid, 10 % Kuralon
getränkt mit einer Lösung von:
19 mmol/l Chlorphenolrot-galactosid
100 mmol/l HEPES pH 7,25
10 mmol/l Borsäure

Flüssigkeitspipettierungen

Als Probe werden in die Probenauftragskammer (PK) 56 µl einer 0,9 Gew.-% NaCl-Lösung pipettiert.

Reaktionsdurchführung

Wenn die Probe in die Probenauftragskammer pipettiert ist, beginnt das Zentrifugationsprogramm. Durch das Zusammenwirken von Schwerkraft, Kapillarkräften und Zentrifugalkraft wird die Probenlösung durch die verschiedenen Reagenzträger hindurch bis in die Küvette transportiert. Um die zeitliche Abfolge der Reaktionsschritte zu steuern, wird das in Beispiel 4 von EP-A 0167171 dargestellte Zentrifugenprogramm verwendet.

Zur Messung wird die Lösung in die Küvette transportiert und die Reaktion absorptions-photometrisch bei 578 nm verfolgt. Die Konjugatmoleküle, welche nicht von der Matrix (Vlies e) gebunden werden, konnten die Matrix passieren und es befindet sich jetzt eine entsprechende Menge Enzym in der Küvette. Die gemessene Farbzunahme pro Zeiteinheit (Extinktion/min, mE/min) ist somit ein Maß für die Bindekapazität der Matrix. Je geringer die gemessene Farbzunahme pro Minute, desto größer ist die Bindekapazität der Matrix.

Fig. 2 zeigt eine Vorrichtung, wie es zur Durchführung von Beispiel 3 verwendet wird. Dabei bedeuten:
PAK: Probenauftragskammer
VK1, VK2, VK3: Ventilkammern
K: Meßküvette
L: Lüftungskanal
Die einzelnen Vliese und Kammern sind schematisch dargestellt. Dabei bedeuten Stege, die den Kontakt zwischen den einzelnen Kammern und Vliesen herstellen, jeweils Verbindungen durch die die zu untersuchende Flüssigkeit weitertransprotiert werden kann.

Die Ergebnisse sind der folgenden Tabelle zu entnehmen:

Tabelle

| Stabilitätsvergleich Haptenbindung mit und ohne Spacer | | | | |
|---|---|---|---|---|
| Spacer | ohne Spacer | niedermolekulare Spacer | hochmolekulare Spacer | |
| Matrix | BOC-$T_3$-Cellulose | BOC-$T_3$-DADOO-TCT-Cellulose | BOC-$T_3$-SPH-TCT-Cellulose | BOC-$T_3$-SPH an Perjodat oxidierte Cellulose |
| Stabilität | + 10 mE/3 Wochen stabil | + 80 mE/3 Wochen instabil | + 80 mE/3 Wochen instabil | + 80 bis 100 mE/3 Wochen instabil |
| Herstellung nach | Beispiel 1 und Beispiel 2 | Beispiel 4 | Beispiel 3 | Beispiel 3 |

## Ansprüche

1. Verfahren zur Herstellung einer Festphasenmatrix, an die Haptene kovalent gebunden sind, **dadurch gekennzeichnet,**
daß man durch Umsetzen eines mindestens eine funktionelle Gruppe enthaltenden Haptens und eines Festphasenmaterials, wobei eine der beiden Komponenten vorher aktiviert wurde, das Hapten an dem als Festphase dienenden Material direkt ohne Bindung eines Spacers kovalent bindet.

2. Verfahren nach Anspruch 1, **dadurch gekennzeichnet,** daß man als Festphase wasserunlösliches Polysaccharid verwendet.

3. Verfahren nach Anspruch 1 oder 2, **dadurch gekennzeichnet,**
daß man ein Hapten verwendet, das als funktionelle Gruppe eine Carboxyl-, Amino-, Aldehyd- oder Hydroxylgruppe aufweist.

4. Verfahren nach einem der vorhergehenden Ansprüche, **dadurch gekennzeichnet,**
daß man als Hapten ein Hormon, Vitamin oder Arzneimittel verwendet.

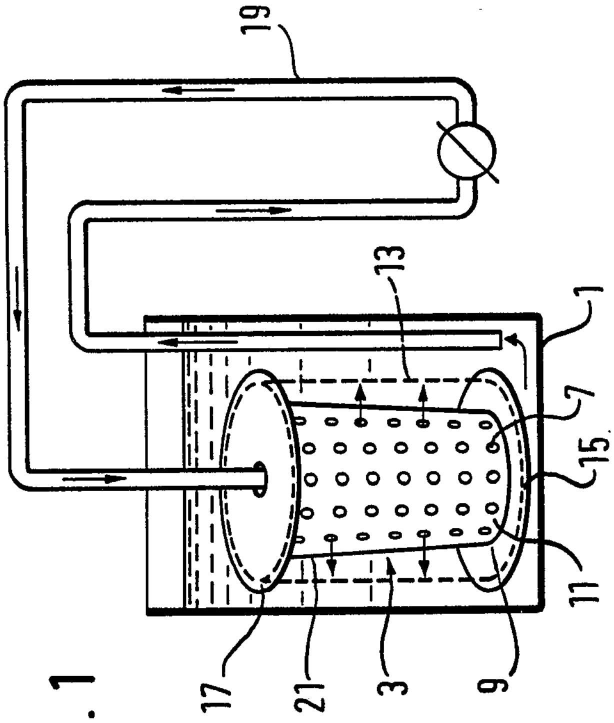


Fig.1

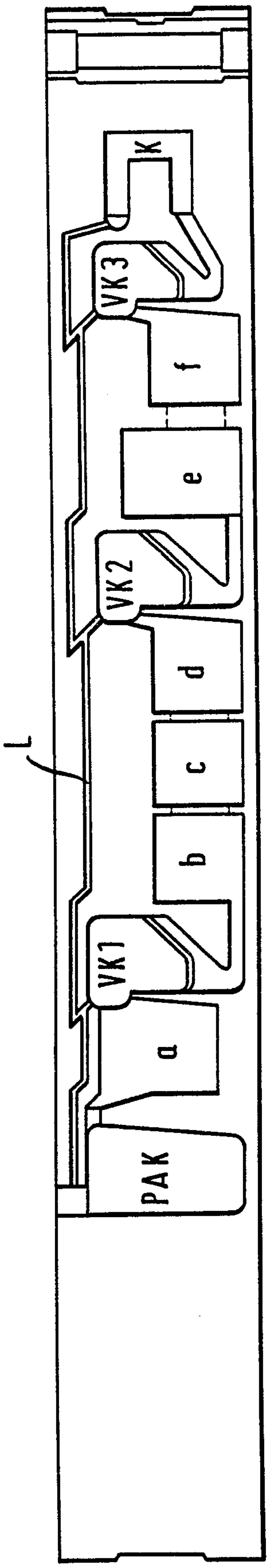


Fig.2